# EUROPÄISCHE PATENTSCHRIFT

(11) **EP 0 799 170 B1**
(45) Veröffentlichungstag und Bekanntmachung des Hinweises auf die Patenterteilung: **08.03.2000**
(21) Anmeldenummer: 95941092.9
(22) Anmeldetag: 12.12.1995
(51) Int. Cl.: C07C 5/42

(54) **VERFAHREN ZUR HERSTELLUNG VON OLEFINISCH UNGESÄTTIGTEN VERBINDUNGEN, INSBESONDERE STYROL DURCH KATALYTISCHE OXIDATION**
METHOD OF PRODUCING OLEFINICALLY UNSATURATED COMPOUNDS, IN PARTICULAR STYRENE, BY CATALYTIC OXIDATION
PROCEDE DE FABRICATION DE COMPOSES OLEFINIQUEMENT INSATURES, NOTAMMENT DE STYRENE, PAR OXYDATION CATALYTIQUE

(30) Priorität: 23.12.1994 DE 4446384
(43) Veröffentlichungstag der Anmeldung: 08.10.1997
(73) Patentinhaber: BASF AKTIENGESELLSCHAFT, 67056 Ludwigshafen (DE)
(72) Erfinder: PÖPEL, Wolfgang, Jürgen, D-64297 Darmstadt (DE); HAGEMEYER, Alfred, D-67063 Ludwigshafen (DE); BÜCHELE, Wolfgang, D-67063 Ludwigshafen (DE); DEIMLING, Axel, D-67434 Neustadt (DE); HOFFMANN, Wolfgang, D-67227 Frankenthal (DE)
(86) Internationale Anmeldenummer: EP9504895
(87) Internationale Veröffentlichungsnummer: WO9620149

(56) Entgegenhaltungen:
- EP-A- 0 403 462
- DE-B- 1 301 810

## Beschreibung

Die Erfindung betrifft ein Verfahren zur Herstellung von olefinisch ungesättigten Verbindungen durch katalytische Sauerstoffübertragung von einem vorher oxidierten, als Katalysator wirkenden Sauerstoffüberträger auf entsprechende Kohlenwasserstoffe (Dehydrierung) in Abwesenheit von molekularem Sauerstoff. Die Erfindung betrifft vorzugsweise die katalytische oxidative Dehydrierung von Alkylaromaten und Paraffinen zu den entsprechenden Alkenylaromaten und Olefinen, insbesondere die Dehydrierung von Ethylbenzol zu Styrol, wobei Wasser gebildet wird.

Styrol und Divinylbenzol sind wichtige Monomere für technische Kunststoffe und werden in großen Mengen gebraucht.

Styrol wird überwiegend durch nicht-oxidative Dehydrierung von Ethylbenzol an modifizierten Eisenoxid-Katalysatoren hergestellt, wobei pro mol Styrol ein mol Wasserstoff entsteht. Nachteiligerweise handelt es sich bei dieser Umsetzung um eine Gleichgewichtsreaktion, die bei Temperaturen von typischerweise 600 bis 700°C abläuft und mit einem Umsatz von ca. 60 % bei einer Styrol-Selektivität von etwa 90 % verläuft, wobei mit steigendem Umsatz und steigender Konzentration des Zielprodukts die Rückreaktion einsetzt und den Umsatz begrenzt.

Durch oxidative Dehydrierung, bei der der umzusetzende Kohlenwasserstoff mit molekularem Sauerstoff umgesetzt wird, läßt sich dagegen ein fast quantitativer Umsatz erzielen, da hierbei Wasser gebildet wird. Auch läuft diese Umsetzung schon bei tieferer Temperatur ab als die nichtoxidative Dehydrierung. Von Nachteil ist bei dieser Verfahrensweise, daß als Nebenreaktion Kohlenoxid und -dioxid gebildet werden (Totaloxidation).

Es ist deshalb vorgeschlagen worden, anstelle von molekularem Sauerstoff einen als Katalysator wirkenden, also reaktionslenkenden, aus einem reduzierbaren Metalloxid bestehenden Sauerstoffüberträger einzusetzen. Dabei erschöpft sich der Sauerstoffüberträger nach und nach und wird in einem zweiten Schritt regeneriert. Dieses in der klassischen Verfahrenstechnik häufig benutzte Prinzip wird als Regenerativprinzip oder instationäre Verfahrensweise bezeichnet. Im Falle der oxidativen Dehydrierung ist die Enthalpiebilanz der Bruttoreaktion, d.h. der Summe der beiden Teilschritte negativ. Durch die Entkopplung von Reduktions- und Oxidationsschritt kann die Selektivität deutlich gesteigert werden.

Das Regenerativprinzip unter Einsatz eines reduzierbaren und regenerierbaren Katalysators wurde zuerst für die Oxidation bzw. Ammonoxidation von Propen zu Acrclein und Acrylsäure bzw. Acrylnitril beschrieben (GB 885 422; GB 999 629; K. Aykan, J. Catal. 12 (1968) 281 - 190), wobei Arsenat- und Molybdatkatalysatoren verwendet wurden. Der Einsatz des Regenerativ-Verfahrens bei der oxidativen Dehydrierung von aliphatischen Alkanen zu Mono- und Diolefinen mit Ferritkatalysatoren (z.B. US 3 440 299, DE 21 18 344, DE 17 93 499) ist ebenfalls bekannt, ebenso wie der Einsatz für die oxidative Kupplung von Methan zu höheren Kohlenwasserstoffen; Katalysatoren unterschiedlichen Aufbaus werden dabei eingesetzt (US 4 795 849, DE 35 86 769 mit Mn/Mg/Si-Oxiden; US 4 568 789 mit Ru-Oxid; EP 254 423 mit Mn/B-Oxiden auf MgO; GB 2 156 842 mit Mn₃O₄-Spinellen). Auch die Dehydrodimerisierung von Toluol zu Stilben in Abwesenheit von freiem Sauerstoff mittels reduzierbarer Katalysatoren wie Bi/In/Ag-Oxiden (EP 30 837) ist bekannt. Schließlich wird das Prinzip noch für die Dehydrierung, Dehydrocyclisierung und Dehydroaromatisierung von Paraffinkohlenwasserstoffen zur Benzinveredelung angewandt (US 4 396 537 mit Co/P-Oxid-Katalysatoren).

Aus den EP 397 37 und 403 462 ist bekannt, das Verfahrensprinzip für die oxidative Dehydrierung von Paraffinkohlenwasserstoffen und Alkylaromaten einzusetzen. Danach werden reduzierbare Metalloxide verwendet, ausgewählt aus der Gruppe V, Cr, Mn, Fe, Co, Pb, Bi, Mo, U und Sn, aufgebracht auf Trägern aus Tonen, Zeolithen und Oxiden von Ti, Zr, Zn, Th, Mg, Ca, Ba, Si, Al.

Die vorstehende Aufzählung ist nur ein Auszug aus dem umfangreichen Schrifttum und betrifft nur die chemisch näherliegenden Verfahren. Ergänzend und ohne näheres Eingehen wird verwiesen auf die folgenden Druckschriften: EP 558 148; EP 556489; EP 543535; US 3 488 402.

Technisch gibt es zwei Varianten der Entkoppelung, nämlich die räumliche und die zeitliche Trennung der beiden Teilschritte.

Bei der räumlichen Trennung der beiden Teilschritte wird ein Wanderbett oder eine zirkulierende Wirbelschicht verwendet, wobei die Katalysatorteilchen aus der Dehydrierzone, nach Abtrennung der Reaktionsprodukte, zu einem separaten Regenerierungsreaktor gefördert werden, in dem die Reoxidation abläuft. Der regenerierte Katalysator wird in die Dehydrierzone zurückgeführt. Ein solches Verfahren kann kontinuierlich, d.h. zyklisch eingerichtet werden. Der Katalysator ist hohen mechanischen Beanspruchungen ausgesetzt und muß daher über eine ausreichende Härte verfügen.

Eine zeitliche Trennung läßt sich mit einem fest angeordneten Sauerstoffüberträger/Katalysator verwirklichen, wobei man periodisch zwischen der Zuführung des Kohlenwasserstoffs, d.h. der Nutzreaktion und der Regenerierung umschaltet.

Die zitierten Dokumente beschreiben Verfahren zur Durchführung instationärer Oxidationen. Die Gewinnung der freiwerdenden Abwärme und die Kopplung mehrerer alternierend betriebener Reaktoren zu einem Wärmeverbund wurde bisher nicht beschrieben.

An sich ist die Einrichtung von Wärmeverbunden, d.h. die wärmetechnische Kopplung zweier unterschiedlicher Reaktionen, von denen eine exotherm und die andere endotherm ist und beide stationär, d.h. ohne Umschalten, betrieben werden, bekannt.

So beschreibt NE-A-93 00 168 die Kombination einer exotherm verlaufenden oxidativen Umsetzung von Methan mit einer nicht näher spezifizierten endothermen Reaktion, wobei Wärmeaustausch immer in derselben Richtung von der ersten zur zweiten Reaktion stattfindet.

Die Erfindung hat sich die Aufgabe gestellt, für ein Verfahren der eingangs genannten Art eine Betriebsweise und apparative Anordnung anzugeben , mit der die Gewinnung der Nettoenthalpie dieses Verfahrens zuverlässig, möglichst wirtschaftlich und mit geringem apparativem Aufwand gelingt.

Eine weitere Aufgabe der Erfindung ist eine möglichst hohe Raum-Zeit-Ausbeute der Nutzreaktion, sowie - durch verbesserte Wärmeführung der Dehydrierung - Verringerung des Nebenprodukt- Anteils und damit ein effizienterer Rohstoffverbrauch.

Unmittelbarer Erfindungsgegenstand ist ein Verfahren der eingangs genannten Art, bei dem mindestens zwei, einen Katalysator für die oxidative Dehydrierung enthaltende und jeweils mindestens einen Wärmeaustauscher aufweisende Reaktoren, in denen die Dehydrierung und Regenerierung zeitlich abwechselnd stattfindet, dadurch zu einem Wärmeverbund gekoppelt werden, daß die Wärmeaustauscher durch einen gemeinsamen Wärmeträgerkreislauf wärmetechnisch miteinander verbunden sind.

Vorteilhaft wird die anfallende Nettoenthalpie dem Wärmeträgerkreislauf an einer geeigneten Stelle in an sich bekannter Weise derart entzogen, daß sie an anderer Stelle nutzbringend verwertet werden kann.

Als Wärmeträger kann ein Wärmeträgergas oder z.B. eine Salzschmelze dienen. Organische Wärmeträger sind wegen des hohen Temperaturniveaus weniger geeignet; ein besonders vorteilhaftes Wärmeträgergas ist Heißdampf, der - z.B durch Einspritzen von Wasser - leicht auf der jeweils bestgeeigneten Temperatur gehalten werden kann, wofür eine umfangreiche Technologie zur Verfügung steht.

Die bei der Regenerierung freigesetzten bzw. bei der Nutzreaktion aufgenommenen Wärmemengen können aus den jeweiligen Reaktionsenthalpien berechnet werden, die ihrerseits aus den Standardbildungsenthalpien zugänglich sind.

Die Teilschritte bei instationärer Fahrweise mit einem sauerstoffübertragenden Redoxkatalysator bestehen aus Dehydrierung (etwa 90% Styrolausbeute) und Regenerierung des Katalysators, wobei (am Beispiel des Systems V₂O₅/V₂O₃) die Dehydrierung endotherm mit einer Bildungsenthalpie von +42.1 kJ/mol bei 500°C verläuft, während die Regenerierung exotherm -164.2 kJ/mol liefert.

Man erkennt hieraus, daß die Nettoenthalpie der oxidativen Dehydrierung eine erhebliche Wärmemenge darstellt, die zudem auf thermodynamisch wünschenswert hohem Niveau zur Verfügung gestellt wird, sodaß die freiwerdende Abwärme nach dem erfindungsgemäßen Verfahren unmittelbar, d.h. ohne etwa Wärmepumpen o.ä. einzusetzen, für die Erzeugung z.B von wertvollem Heißdampf und den Antrieb von modernen Stromerzeugungsaggregaten genutzt werden kann.

Außerdem ist von Vorteil, daß die endotherme Nutzreaktion, also die Dehydrierung beispielsweise von Ethylbenzol bei einer um rund 100° niedrigeren Temperatur abläuft als die exotherme Regenerierung, sodaß man es leicht einrichten kann, die nach dem Entzug der Nettoenthalpie verbleibende Restwärme auf genau dem benötigten Temperaturniveau zum Betrieb der endothermen Nutzreaktion zur Verfügung zu stellen.

In einer geeigneten Anordnung werden mehrere Reaktoren (deren Gesamtzahl sei n) zu einem Verbund zusammengeschaltet derart, daß sich im gegenseitigen Wechselbetrieb eine gewisse Zahl von Reaktoren (m Reaktoren, wobei m < n sei) in der Dehydrierphase befinden, während sich gleichzeitig die übrigen n-m Reaktoren in der Regenerierphase befinden. Beim Umschaltvorgang werden dann eine jeweils erforderliche Anzahl Reaktoren von Dehydrierung auf Regenerierung und ebensoviele Reaktoren von Regenerierung auf Dehydrierung geschaltet.

Die Teilschritte Dehydrierung und Reoxidation können zeitlich entkoppelt werden, derart, daß unter Verwendung eines Katalysatorfestbettes ein periodisches Umschalten des Reaktoreingangsstromes zwischen den Edukten und dem Regeneriergas stattfindet.

Zwischen dem Dehydrierschritt und dem Regenerierschritt kann eine Spülphase eingefügt werden, in der der Festbettreaktor von einem Spülgas durchströmt wird.

Bevorzugt sind die zu dehydrierenden Kohlenwasserstoffe Alkylaromaten, die zu den entsprechenden Alkenylaromaten dehydriert werden.

Besonders bevorzugt wird Ethylbenzol durch katalytische oxidative Dehydrierung mittels eines Redoxkatalysators zu Styrol dehydriert.

Im einfachsten Fall verwendet man zwei alternierend betriebene Festbettreaktoren. Im Wälzgas- bzw. Salzschmelzekreislauf, der für die Wärmezufuhr zum Reaktor in der momentanen Dehydrierphase und die Wärmeabfuhr aus dem Reaktor in der momentanen Regenerierphase sorgt, ist ein Wärmetauscher vorgesehen, mit dem Dampf erzeugt und in das Netz eines größeren Dampf-Verbundes abgegeben werden kann. Beim Phasenwechsel wird der bisherige Dehydrierreaktor regeneriert und der andere Reaktor für die Nutzreaktion eingesetzt. Dabei ist zunächst vorausgesetzt, daß etwa gleich lange Dehydrier- und Regenerierzeiten erforderlich sind.

Natürlich können auch mehr als 2 Reaktoren zu einem Verbund zusammengeschaltet werden. Verläuft etwa die Regenerierung langsamer als die Dehydrierung, so können beispielsweise 3 Reaktoren im Wechselbetrieb so gekoppelt werden, daß in einem Reaktor dehydriert wird, während in den anderen 2 Reaktoren regeneriert wird und die Kette dann beim nächsten Phasenwechsel jeweils um einen Reaktor weitergeschaltet wird. Ist umgekehrt die Regenerierung schneller als die Dehydrierung, so wird man einen Reaktor in der Regenerationsphase betreiben und die anderen beiden in der Dehydrierphase. Die relativen Geschwindigkeiten von Dehydrierung und Regenerierung hängen von der chemischen Natur des jeweiligen Rohstoffs und der gewählten Reaktionstemperatur ab. Grundsätzlich gilt, daß sich die erforderlichen Dehydrier- und Regenerierperioden umso besser aufeinander abstimmen und damit die Raum-Zeit-Ausbeute optimieren läßt, je mehr Reaktoren zur Verfügung stehen.

Technisch eingeführte Oxidations/Dehydrierungsverfahren werden bei Temperaturen von 100 bis 900°C, bevorzugt 200 bis 800°C, insbesondere 250 bis 750°C und bei Drücken von 100 mbar bis 10 bar, bevorzugt 500 mbar bis 2 bar, mit einer, auf flüssigen Aggregatzustand (liquid hourly space velocity; LHSV) bezogenen LHSV von 0.01 bis 20 h⁻¹, bevorzugt 0.1 bis 5 h⁻¹ durchgeführt. Neben dem zu oxidierenden/dehydrierenden Kohlenwasserstoff können Verdünnungsmittel wie beispielsweise CO₂, N₂, Edelgase oder Dampf zugegen sein.

Speziell für die oxidative Dehydrierung von Verbindungen vom Typ des Styrols besteht ein geeigneter Katalysator zweckmäßig aus wenigstens einem reduzierbaren Metalloxid, ausgewählt aus der Gruppe der Oxide von Ce, Cr, V, Mn, Fe, Co, Nb, Mo, Wo, In, Cu, Ag, Sn, Pb, Sb oder Bi das sowohl ungeträgert als auch auf einen Träger aufgebracht sein kann, der ausgewählt sein kann aus der Gruppe der Tone, aufgeweiteten Tone (PILC; pillared clay), Zeolithe, Aluminiumphosphate, SiC, Si₃N₄, BN, C (Graphit), und/oder der Metalloxide ausgewählt aus der Gruppe der Oxide von Ti, Zr, Zn, Th, Mg, Ca, Ba, Si, Al, und noch Promotoren, insbesondere (Erd-)Alkali und/oder Seltene Erden enthalten kann.

Die Regenerierung des erschöpften Katalysators wird bei Temperaturen im Bereich 100 bis 800°C, bevorzugt 250 bis 600°C mit einem freien Oxidationsmittel, vorzugsweise mit N₂O oder einem sauerstoffhaltigen Gas einschließlich reinem Sauerstoff , durchgeführt. Auch hier können Verdünnungsmittel im Regenerierstrom enthalten sein. Geeignete Regeneriergase sind z.B. Luft, Magerluft, Sauerstoff oder N₂O. Die Regenerierung kann bei subatmosphärischem, atmosphärischem oder überatmosphärischem Druck betrieben werden. Bevorzugt sind Drücke im Bereich 500 mbar bis 10 bar.

Die Verweilzeit bei der Dehydrierung kann z.B. im Bereich von 0.01 bis 20 Sekunden, vorzugsweise 0.05 bis 5 und besonders bevorzugt im Bereich von 0.1 bis 3 Sekunden liegen.

Die Verweilzeit bei der Regenerierung kann z.B. im Bereich von 0,01 bis 100 Sekunden, vorzugsweise 0.05 bis 50 und besonders bevorzugt im Bereich von 0,1 bis 30 Sekunden liegen.

Alle üblichen, nach beliebigen Methoden hergestellten und für die Anordnung im Festbett oder evt. auch zur Anordnung in einer Wirbelschicht geeigneten Katalysatoren können für das erfindungsgemäße Verfahren verwendet werden. Bevorzugte Katalysatoren bestehen aus wenigstens einem reduzierbaren Bismuth- und/oder Cer- und/oder Vanadium-Katalysator, der evt. auf einem Träger, z.B. auf der Grundlage von -Titan- und/oder Chrom-Oxid aufgetragen und evt. noch mit einem Alkali- Erdalkali- und/oder Selten-Erdmetall promotiert sein kann.

### Beispiel 1

Zur Herstellung eines Katalysators, der Vanadium auf einem Magnesium-Oxid-Träger enthalten soll, werden 336,3 g Ammonium-meta-vanadat und 900 g Magnesiumoxid in 8 l Wasser eingerührt und das Rühren für eine Stunde fortgesetzt. Das in einem Sprühtrockner gewonnene Pulver wird mit wenig Wasser und einem Verstrangungshilfsmittel angeteigt, für 2 Stunden in einem Laborkneter bearbeitet und danach zu 3mm-Strängen verformt, die für 16 Stunden bei 120°C getrocknet, bei 600°C calciniert und zerkleinert wurden. Dieser Katalysator enthält 22,5 % V₂O₅ und 77.5 % MgO. Für die Versuche wurde eine Splitt-Fraktion von 0,5 bis 0,7 mm ausgesiebt.

In einem Labor-Wendelreaktor werden 9,3 g des Katalysators vorgelegt, sodaß das eingefüllte Katalalysatorvolumen 20 ml beträgt. Der Reaktor befindet sich zur Temperierung in einer auf 500°C gehaltenen Salzschmelze. Der den Reaktor verlassende Gasstrom kann kondensiert, aufgefangen und mittels GC untersucht werden.

Mit einer Förderpumpe werden stündlich 10 ml Ethylbenzol aus einem Vorratsbehälter entnommen und in einem Stickstoffstrom so verdampft, das der Stickstoff-Fluß (zur Einregulierung der Verweilzeit) 8 bis 28 Nl/h beträgt. Die Verweilzeit ergibt sich daraus zwischen 0,8 und 3 Sekunden. Wie man leicht errechnet, beträgt LHSV 0,5 h⁻¹.

Dehydrier- und Regenerierphase dauern jeweils 5 Minuten, d.h. ein vollständiger Zyklus 10 Minuten.

Bei Verwendung von nur einem Reaktor im Salzbad werden pro Cyclus 0,51 g Styrol erhalten, entsprechend einer Raum-Zeit-Ausbeute (RZA) von 0,33 kg Styrol je kg Katalysator und Stunde. Bei entsprechender Verdoppelung der Versuchsanlage und Betrieb im erfindungsgemäßen Verbund wird eine RZA von 0,41 kg Styrol je kg Katalysator und Stunde erreicht.

### Beispiel 2

In einer Laboranlage wurde bei einer LHSV von 0.5 h⁻¹ die isotherme oxidative Dehydrierung von Ethylbenzol zu Styrol bei 500°C durchgeführt. Dabei wird der in Beispiel 1 beschriebene Katalysator während der Dehydrierung zunächst reduziert und nach Wechsel des Zulaufs auf Luft reoxidiert. Die Zykluszeit (Reduktions- und Oxidationsschritt) beträgt 10 Minuten. Bei einem Umsatz von 95 % Ethylbenzol wurde eine Selektivität von 95 % erreicht.

Bei der Laboranlage beträgt die überschüssige (und somit theoretisch ausschleusbare Wärmemenge) nach Abzug der schwach endothermen oxidativen Dehydrierung (incl. Katalysatorreduktion) 1.7 W.

Vergleichsversuch: Bei der nicht oxidativen Dehydrierung von Ethylbenzol zu Styrol wird bei etwa 600°C und einem Ethylbenzolumsatz von 60% eine Selektivität von 94% erzielt. Die RZA beträgt etwa 0.2.

## Patentansprüche

1. Verfahren zur Herstellung von olefinisch ungesättigten Verbindungen durch katalytische Sauerstoffübertragung von einem vorher oxidierten, als Katalysator wirkenden Sauerstoffüberträger auf entsprechende Kohlenwasserstoffe (Dehydrierung) in Abwesenheit von molekularem Sauerstoff und Regenerierung (Reoxidation) des Sauerstoffüberträgers, dadurch gekennzeichnet, daß mindestens zwei, jeweils einen Katalysator für die oxidative Dehydrierung enthaltende und jeweils mindestens einen Wärmeaustauscher aufweisende Reaktoren, in denen die Dehydrierung und Regenerierung zeitlich abwechselnd stattfindet, dadurch zu einem Wärmeverbund gekoppelt werden, daß die Wärmeaustauscher durch einen gemeinsamen Wärmeträgerkreislauf wärmetechnisch miteinander verbunden sind.

2. Verfahren nach Anspruch 1, dadurch gekennzeichnet, daß als Wärmeüberträger ein Wälzgas oder eine Salzschmelze verwendet werden.

3. Verfahren nach Anspruch 1, dadurch gekennzeichnet, daß dem Wärmeüberträgerkreislauf eine Nettowärmemenge entzogen und außerhalb des Verfahrens eingesetzt wird.

4. Verfahren nach Anspruch 1, dadurch gekennzeichnet, daß die Teilschritte Dehydrierung und Reoxidation zeitlich entkoppelt sind, derart, daß unter Verwendung eines Katalysatorfestbettes ein periodisches Umschalten des Reaktoreingangsstromes zwischen den Edukten und dem Regeneriergas stattfindet.

5. Verfahren nach Anspruch 1, dadurch gekennzeichnet, daß zwischen dem Dehydrierschritt und dem Regenerierschritt eine Spülphase eingefügt wird, in der der Festbettreaktor von einem Spülgas durchströmt wird.

6. Verfahren nach Anspruch 1, dadurch gekennzeichnet, daß die zu dehydrierenden Kohlenwasserstoffe Alkylaromaten sind, die zu den entsprechenden Alkenylaromaten dehydriert werden.

7. Verfahren nach Anspruch 6, dadurch gekennzeichnet, daß Ethylbenzol durch katalytische oxidative Dehydrierung mittels eines Redoxkatalysators zu Styrol dehydriert wird.

8. Verfahren nach Anspruch 1, dadurch gekennzeichnet, daß der reduzierte Katalysator mit einem sauerstoffhaltigen Gas oder reinem Sauerstoff regeneriert wird.

9. Verfahren nach Anspruch 1, dadurch gekennzeichnet, daß der reduzierte Katalysator mit N₂O als Oxidationsmittel regeneriert wird.

10. Verfahren nach Anspruch 1, dadurch gekennzeichnet, daß ein Katalysator verwendet wird, der mindestens ein reduzierbares Metalloxid, ausgewählt aus der Gruppe der Oxide von Ce, Cr, V, Mn, Fe, Co, Nb, Mo, Wo, In, Cu, Ag, Sn, Pb, Sb oder Bi trägerfrei oder auf einem Träger angeordnet enthält.

11. Verfahren nach Anspruch 10, dadurch gekennzeichnet, daß der Katalysator auf einen Träger angeordnet ist, ausgewählt aus der Gruppe der Tone, aufgeweiteten Tone (PILC; pillared clay), Zeolithe, Aluminiumphosphate, Siliciumnitrid und -carbid, Bornitrid und -carbid, Graphit und/oder der Metalloxide ausgewählt aus der Gruppe der Oxide von Ti, Zr, Zn, Th, Mg, Ca, Ba, Si, Al, aufgebracht ist und noch Promotoren, insbesondere (Erd-)Alkali und/oder Seltene Erden enthalten kann.

12. Verfahren nach Anspruch 11, dadurch gekennzeichnet, daß der Katalysator wenigstens ein reduzierbares Oxid von Bismuth, Vanadium oder Cer auf einem Titanoxid- oder Chromoxidträger enthält.

13. Verfahren nach Anspruch 10, 11 oder 12, dadurch gekennzeichnet, daß der Katalysator einen Promotor aus der Gruppe der Alkali-, Erdalkali- und/oder Seltenerdmetalle enthält.

14. Verfahren nach Anspruch 10, 11 oder 12, dessen dehydrierender Teilschritt im Temperaturbereich zwischen 200 und 800°C, bei einem Druck von 100 mbar bis 10 bar und mit einer LHSV von 0.01 bis 20 h⁻¹ ausgeführt wird.

## Claims

1. A process for preparing olefinically unsaturated compounds by catalytic oxygen transfer from a previously oxidized oxygen transferer acting as catalyst to corresponding hydrocarbons (dehydrogenation) in the absence of molecular oxygen and regeneration (reoxidation) of the oxygen transferer, wherein at least two reactors each containing a catalyst for the oxidative dehydrogenation and each having at least one heat exchanger, in which reactors the dehydrogenation and regeneration takes place alternately in time, are coupled to form an integrated heat system by the heat exchangers being connected to one another in terms of heat by means of a common circuit for heat transfer medium.

2. A process as claimed in claim 1, wherein the heat transfer medium used is a circulating gas or a salt melt.

3. A process as claimed in claim 1, wherein a net amount of heat is drawn from the circuit for heat transfer medium and is used outside the process.

4. A process as claimed in claim 1, wherein the sub-steps dehydrogenation and reoxidation are decoupled in time in such a way that, using a fixed catalyst bed, the reactor inlet stream is periodically switched between the starting materials and the regeneration gas.

5. A process as claimed in claim 1, wherein between the dehydrogenation step and the regeneration step there is inserted a flushing phase in which a flushing gas flows through the fixed bed reactor.

6. A process as claimed in claim 1, wherein the hydrocarbons to be dehydrogenated are alkylaromatics which are dehydrogenated to give the corresponding alkenylaromatics.

7. A process as claimed in claim 6, wherein ethylbenzene is dehydrogenated by catalytic oxidative dehydrogenation using a redox catalyst to give styrene.

8. A process as claimed in claim 1, wherein the reduced catalyst is regenerated using an oxygen-containing gas or pure oxygen.

9. A process as claimed in claim 1, wherein the reduced catalyst is regenerated using N₂O as oxidant.

10. A process as claimed in claim 1, wherein the catalyst used comprises at least one reducible metal oxide selected from the group of the oxides of Ce, Cr, V, Mn, Fe, Co, Nb, Mo, Wo, In, Cu, Ag, Sn, Pb, Sb or Bi, either unsupported or on a support.

11. A process as claimed in claim 10, wherein the catalyst is applied to a support selected from the group of clays, pillared clays (PILC), zeolites, aluminum phosphates, silicon nitride and carbide, boron nitride and carbide, graphite and/or the metal oxides selected from the group of the oxides of Ti, Zr, Zn, Th, Mg, Ca, Ba, Si, Al, and can additionally comprise promoters, in particular alkali metals, alkaline earth metals and/or rare earths.

12. A process as claimed in claim 11, wherein the catalyst comprises at least one reducible oxide of bismuth, vanadium or cerium on a titanium oxide or chromium oxide support.

13. A process as claimed in claim 10, 11 or 12, wherein the catalyst comprises a promoter selected from the group of alkali, alkaline earth and/or rare earth metals.

14. A process as claimed in claim 10, 11 or 12, whose dehydrogenating sub-step is carried out at from 200 to 800°C, at a pressure of from 100 mbar to 10 bar and at an LHSV of from 0.01 to 20 h⁻¹.

## Revendications

1. Procédé pour la préparation de composés à insaturation oléfinique par transfert catalytique d'oxygène d'un agent de transfert de l'oxygène oxydé au préalable agissant en tant que catalyseur sur les hydrocarbures appropriés (déshydrogénation) en l'absence d'oxygène moléculaire et régénération (réoxydation) de l'agent de transfert de l'oxygène, caractérisé par le fait que l'on couple en un ensemble thermique au moins deux réacteurs contenant chacun un catalyseur de la déshydrogénation oxydative et équipé chacun d'au moins un échangeur de chaleur, et dans lesquels on procède alternativement à la déshydrogénation et à la régénération, et que les échangeurs de chaleur sont en liaison thermique entre eux par un circuit commun d'agent de transfert de la chaleur.

2. Procédé selon la revendication 1, caractérisé par le fait que l'on utilise en tant qu'agent de transfert de la chaleur, un gaz en circulation ou une masse de sels fondus.

3. Procédé selon la revendication 1, caractérisé par le fait que l'on retire une quantité de chaleur nette du circuit de transfert de la chaleur et on l'exploite en dehors du procédé.

4. Procédé selon la revendication 1, caractérisé par le fait que les stades partiels de déshydrogénation et de réoxydation sont découplés dans le temps en ce que, avec utilisation d'un lit fixe de catalyseur, il se produit une permutation périodique du courant d'entrée dans le réacteur entre les composants de départ et le gaz régénéré.

5. Procédé selon la revendication 1, caractérisé par le fait que, entre le stade de déshydrogénation et le stade régénération, on procède à une phase de balayage dans laquelle le réacteur à lit fixe est traversé par un gaz de balayage.

6. Procédé selon la revendication 1, caractérisé par le fait que les hydrocarbures soumis à déshydrogénation sont des hydrocarbures alkylaromatiques qui sont déshydrogénés en les hydrocarbures alcénylaromatiques correspondants.

7. Procédé selon la revendication 6, caractérisé par le fait que l'on convertit l'éthylbenzène en styrène par déshydrogénation oxydative catalytique sur un catalyseur rédox.

8. Procédé selon la revendication 1, caractérisé par le fait que le catalyseur réduit est régénéré à l'aide d'un gaz contenant de l'oxygène ou à l'aide d'oxygène pur.

9. Procédé selon la revendication 1, caractérisé par le fait que le catalyseur réduit est régénéré à l'aide de N₂O servant d'agent oxydant.

10. Procédé selon la revendication 1, caractérisé par le fait que l'on utilise un catalyseur qui contient au moins un oxyde métallique réductible choisi dans le groupe des oxydes de Ce, Cr, V, Mn, Fe, Co, Nb, Mo, Wo, In, Cu, Ag, Sn, Pb, Sb ou Bi, sans support ou appliqué sur un support.

11. Procédé selon la revendication 10, caractérisé par le fait que le catalyseur est appliqué sur un support choisi dans le groupe des argiles, des argiles dilatées (PILC ; pillared clay), des zéolites, des aluminophosphates, du nitrure et du carbure de silicium, du nitrure et du carbure de bore, du graphite et/ou des oxydes métalliques choisis dans les groupes des oxydes de Ti, Zr, Zn, Th, Mg, Ca, Ba, Si, Al, et peut encore contenir des activateurs, en particulier des métaux alcalins et/ou alcalino-terreux et/ou des terres rares.

12. Procédé selon la revendication 11, caractérisé par le fait que le catalyseur contient au moins un oxyde réductible du bismuth, du vanadium ou du cérium sur un support consistant en oxyde de titane ou oxyde de chrome.

13. Procédé selon la revendication 10, 11 ou 12, caractérisé par le fait que le catalyseur contient un activateur du groupe des métaux alcalins, des métaux alcalino-terreux et/ou des métaux des terres rares.

14. Procédé selon la revendication 10, 11 ou 12, dont le stade déshydrogénation est réalisé dans l'intervalle de température de 200 à 800°C, sous une pression de 100 mbar à 10 bar et à une VSHL de 0,01 à 20 h⁻¹.
